# FASCICULE DE BREVET EUROPEEN

(11) **EP 0 695 146 B1**
(45) Date de publication et mention de la délivrance du brevet: **04.06.1997**
(21) Numéro de dépôt: 94913664.2
(22) Date de dépôt: 19.04.1994
(51) Int. Cl.: A61B 17/58

(54) **PIECE D'ASSEMBLAGE POUR DISPOSITIF D'OSTEOSYNTHESE**
AUFBAUTEIL FÜR EINE OSTEOSYNTHETISCHE VORRICHTUNG
MOUNTING MEMBER FOR AN OSTEOSYNTHESIS DEVICE

(30) Priorité: 20.04.1993 FR 9304625
(43) Date de publication de la demande: 07.02.1996
(73) Titulaire: STRYKER CORPORATION, Kalamazoo, MI 49002 (US); HENRY, Patrick, F-92200 Neuilly (FR); LAPRESLE, Philippe, F-92200 Neuilly-sur-Seine (FR); MISSENARD, Gilles, 75016 Paris (FR)
(72) Inventeur: HENRY, Patrick, F-92200 Neuilly-sur-Seine (FR); LAPRESLE, Philippe, F-92200 Neuilly-sur-Seine (FR); MISSENARD, Gilles, F-75016 Paris (FR)
(74) Mandataire: Le Forestier, Eric
(86) Numéro de dépôt international: FR9400437
(87) Numéro de publication internationale: WO9423661

(56) Documents cités:
- CH-A- 408 381
- DE-A- 2 834 891
- FR-A- 2 656 214
- GB-A- 2 033 758

## Description

La présente invention concerne le domaine des pièces d'assemblage utilisées en osthéosynthèse, notamment du rachis et a trait plus particulièrement à une pièce destinée à l'assemblage de deux tiges entre elles.

On connait par la publication EP-A-0 383 992 une pièce d'assemblage utilisée en osthéosynthèse du rachis et servant à relier transversalement deux tiges longitudinales fixées en plusieurs points de leur longueur aux vertèbres. Cette pièce comporte deux éléments identiques formant pince, à assembler sur une tige longitudinale l'un contre l'autre au moyen d'une tige filetée et de deux écrous. Les pièces d'assemblage sont positionnées par paires sur les tiges longitudinales et les deux tiges filetées assemblant leurs éléments sont réunies par un manchon taraudé pour relier transversalement les tiges longitudinales. La mise en place des pièces d'assemblage utilise ainsi un grand nombre d'éléments différents (écrous, tiges filetées, éléments formant pince, manchon taraudé) dont l'assemblage est fastidieux. Par ailleurs ces différents éléments présentent des angles saillants risquant d'endommager les tissus environnants.

La présente invention vise à remédier à ces inconvénients et a pour objet une pièce d'assemblage perfectionnée dont la mise en place est aisée et rapide et qui ne risque pas d'endommager les tissus environnants.

Selon une première caractéristique de l'invention, cette pièce d'assemblage perfectionnée comporte :
. un corps de forme générale arrondie présentant une fente séparant deux parties du corps et deux alésages d'axes transversaux coupant obliquement la fente, adaptés à recevoir chacun une tige à assembler, et
. une vis de serrage reliant lesdites parties de sorte que le serrage de la vis immobilise simultanément les deux tiges par pinçage entre lesdites parties.

Dans un mode de réalisation avantageux de l'invention, ledit corps présente la forme générale d'une sphère, lesdits axes sont orthogonaux, et lesdites parties sont reliées entre elles par un pont de matière délimité extérieurement par un méplat et intérieurement par le fond de la fente, ce dernier s'étendant parallèlement au méplat. Toujours dans un mode de réalisation avantageux de l'invention, la vis comporte une tête destinée à venir en butée axialement contre un épaulement ménagé sur l'une des parties et la vis comporte un filetage destiné à venir en prise avec un taraudage ménagé dans l'autre partie.

D'autres caractéristiques et avantages de la présente invention apparaîtront à la lecture de la description qui va suivre, d'un exemple de réalisation non limitatif de l'invention et à l'examen du dessin annexé sur lequel :

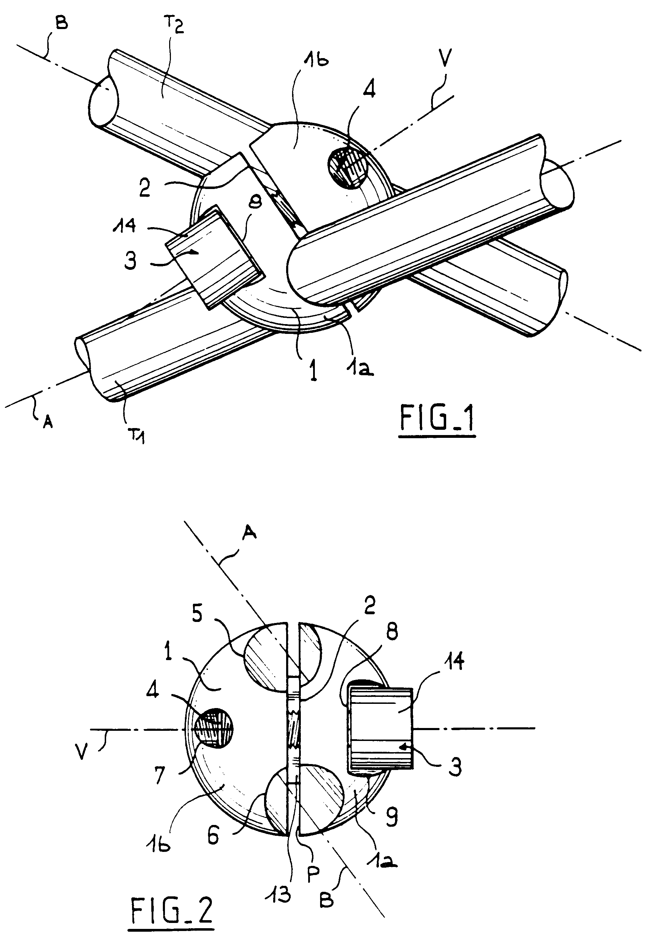

- la figure 1 est une vue en perspective d'une pièce d'assemblage selon l'invention, traversée par deux tiges à assembler,
- la figure 2 est une vue de dessus de la pièce représentée isolément reposant par son méplat sur une surface plane,

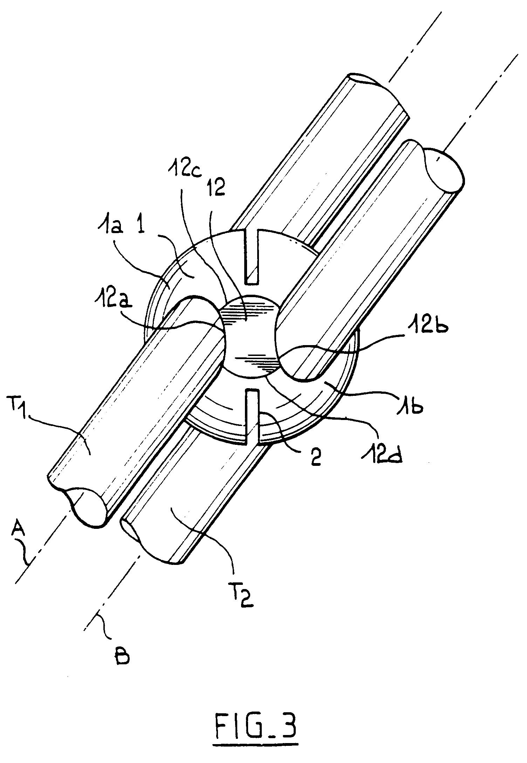

- la figure 3 est une vue de dessous de la pièce d'assemblage traversée par les deux tiges,

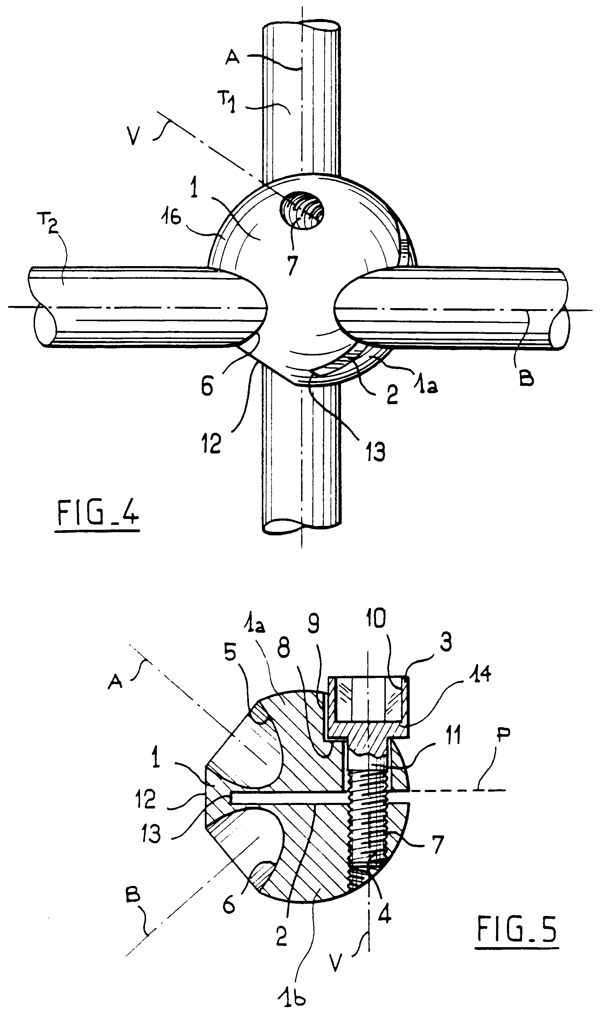

- la figure 4 est une vue prise selon une direction orthogonale aux axes des tiges, et
- la figure 5 est une section de la pièce d'assemblage selon un plan de coupe contenant l'axe de la vis et perpendiculaire au méplat.

La pièce d'assemblage représentée sur les figures est réalisée a partir d'un corps plein 1 constitué de préférence par un alliage de titane. La surface externe du corps 1 est généralement sphérique tronquée et présente un méplat 12. Le corps 1 est fendu selon un plan équatorial P perpendiculaire au méplat 12 pour former une fente 2 séparant deux parties généralement hémisphériques 1a et 1b reliées par un pont de matière 1c s'étendant entre le méplat 12 et le fond 13 de la fente 2. De préférence, comme représenté, la fente 2 présente une épaisseur constante et le fond 13 est plan et parallele au méplat 12.

La pièce d'assemblage est alésée pour recevoir une premiere tige T₁. L'alésage 5 ainsi formé traverse le corps 1 selon un axe A coupant obliquement le plan P et le plan tangent au méplat 12. L'alésage 5 debouche à une extrémité sur la surface externe de la partie 1a en intersectant le méplat 12 selon un arc elliptique 12a et à l'autre extrémité de part et d'autre du plan P sur la surface externe des parties 1a et 1b.

Le corps 1 est également alésé pour recevoir une deuxième tige T₂ à assembler avec la première T₁. L'alésage 6 ainsi formé traverse le corps 1 selon un axe B orthogonal à l'axe A et coupant obliquement le plan P et le plan tangent au méplat 12. L'alésage 6 débouche à une extrémité sur la surface externe de la partie 1b en intersectant le méplat 12 selon un arc elliptique 12b et à l'autre extrémité de part et d'autre du plan P sur la surface externe des parties 1a et 1b. Les arcs elliptiques 12a et 12b sont sensiblement symétriques l'un de l'autre par rapport au plan P et sont réunis à leurs extrémités par des arcs de cercle opposés 12c et 12d sensiblement égaux.

Conformément à l'invention, les deux parties 1a et 1b sont reliées par une vis de serrage 3 de sorte que le serrage de cette dernière immobilise les deux tiges T₁ et T₂ par pinçage entre les parties 1a et 1b. De préférence, comme représenté, la vis 3 s'étend selon un axe V perpendiculaire au plan P et l'axe V est contenu dans un plan équatorial de la sphère perpendiculaire au méplat 12. L'axe V se situe dans celui des deux hémisphères, délimités par le plan équatorial parallèle au méplat 12, qui est à l'opposé de celui portant le méplat 12. Plus précisément, l'axe V passe sensiblement par le milieu du rayon joignant le sommet de cet hémisphère et le plan équatorial précité.

La partie 1a est alésée de façon étagée selon l'axe V pour former un épaulement 8 et la vis 3 comporte une tête 14 venant axialement en butée contre cet épaulement 8. La partie 1b est taraudée en 7 selon l'axe V pour recevoir le filetage 4 de la vis 3. Ce filetage 4 se raccorde par une partie cylindrique non filetée 11 sur la tranche de la tête 14 de la vis venant en butée contre l'épaulement 8.

Le serrage de la vis 3 provoque le fléchissement du pont de matière 1c autour d'un axe parallèle à l'axe V et le rapprochement des parties 1a et 1b, bloquant les tiges T₁ et T₂ en position par pinçage entre les parties 1a et 1b. La flexibilité du pont de matière 1c dépend notamment de son épaisseur et grâce au méplat 12 et à la fente 2 à fond 13 plat parallèle au méplat 12 on obtient une épaisseur constante de matière et l'absence de points de faiblesse localisés.

Les alésages 5 et 6 débouchent sur la fente 2 à une extrémité sensiblement symétriquement de part et d'autre du plan contenant l'axe V et perpendiculaire au méplat 12, de sorte que les tubes T₁ et T₂ sont pincés avec une intensité égale lors du serrage de la vis 3, ce dernier s'effectuant au moyen d'une clé à pans hexagonaux engagée dans un logement 10 de forme complémentaire ménagé sur la face frontale de la tête 14 de la vis.

Dans l'exemple de réalisation décrit, le diamètre de la sphère délimitant le corps 1 vaut 15 mm environ. La fente 2 présente une épaisseur de 1 mm et sa profondeur vaut 13 mm environ. L'épaisseur du pont de matière 1c mesurée entre le fond 13 de la fente et le méplat 12 vaut approximativement 1,5 mm. La vis de serrage 3 présente une longueur de 15 mm, le diamètre du filetage 4 vaut 3 mm et ce dernier s'étend sur une longueur de 8 mm. Le diamètre de la tête 14 vaut 6 mm. L'épaulement 8 s'étend dans un plan situé à mi-distance environ du plan P et du plan contenant le sommet de la surface hémisphérique délimitant la partie 1a et parallèle au plan P de sorte que la tête 14 de la vis 3 vient se loger en majeure partie dans le logement cylindrique 9, de révolution autour de l'axe V et dont le fond constitue l'épaulement 8. Le filetage 4 ne fait que légèrement saillie hors du taraudage 7 et la pièce d'assemblage ne présente pas d'angles saillants risquant d'endommager les tissus environnants,

La pièce d'assemblage selon l'invention est avantageusement destinée au montage de tiges transverses entre deux tiges longitudinales, ancrées en plusieurs points de leur longueur dans les vertèbres au moyen d'implants connus en eux-mêmes. Les pièces d'assemblage sont enfilées grâce à l'un des alésages sur les tiges longitudinales et positionnées par paires sur celles-ci pour recevoir grâce à l'autre alésage une tige transverse. Avantageusement, les deux alésages d'une pièce présentent des diamètres égaux de sorte que l'on peut utiliser comme tige transverse une tige de même nature que les tiges longitudinales. Après positionnement correct des tiges transverses, on procède au serrage des vis 3.

La présente invention n'est pas limitée à l'exemple de réalisation qui vient d'être décrit. On peut notamment proposer de réaliser la pièce d'assemblage dans d'autres matériaux biologiquement compatibles.

## Revendications

1. Pièce d'assemblage destinée à assembler deux tiges (T₁, T₂) d'un dispositif d'osthéosynthèse, notamment d'osthéosynthèse du rachis, caractérisée en ce qu'elle comporte :
. un corps (1) en un matériau biologiquement compatible de forme générale arrondie présentant une fente (2) séparant deux parties (1a, 1b) du corps et deux alésages (5, 6) d'axes (A, B) transversaux coupant obliquement la fente, adaptés à recevoir chacun une tige à assembler, et
. une vis de serrage (3) reliant lesdites parties de sorte que le serrage de la vis immobilise les deux tiges par pinçage entre lesdites parties (1a, 1b).

2. Pièce d'assemblage selon la revendication 1, caractérisée en ce que ledit corps (1) présente la forme générale d'une sphère.

3. Pièce d'assemblage selon l'une des revendications 1 et 2, caractérisée en ce que lesdits axes (A, B) sont orthogonaux.

4. Pièce d'assemblage selon l'une des revendications 1 à 3, caractérisée en ce que lesdites parties (1a, 1b) sont reliées entre elles par un pont de matière (1c) présentant extérieurement un méplat (12) et en ce que le fond (13) de la fente s'étend parallèlement au plan du méplat.

5. Pièce d'assemblage selon l'une des revendications 1 à 4, caractérisée en ce que la vis (3) comporte une tête de vis (14) destinée à venir en butée axialement contre un épaulement ménagé sur l'une (1a) des parties et en ce que la vis comporte un filetage (4) destiné à venir en prise avec un taraudage (7) ménagé sur l'autre partie (1b).

6. Pièce d'assemblage selon la revendication 5, caractérisée en ce que la vis s'étend selon un axe (V) orienté perpendiculairement à la fente (2).

## Claims

1. An assembly piece for assembling together two rods (T₁, T₂) of an ostheosynthesis device, in particular for ostheosynthesis of the spine, the assembly piece being characterized in that it comprises:
• a body (1) of biologically compatible material and of generally rounded shape having a slot (2) separating the body into two portions (1a, 1b), and two bores (5, 6) on transverse axes (A, B) passing obliquely through the slot, each adapted to receive a respective rod to be assembled; and
• a clamping screw (3) interconnecting said portions so that tightening the screw locks the two rods in place by clamping them between said portions (1a, 1b).

2. An assembly piece according to claim 1, characterized in that said body (1) is generally spherical in shape.

3. An assembly piece according to claim 1 or 2, characterized in that said axis (A, B) are orthogonal.

4. An assembly piece according to any one of claims 1 to 3, characterized in that said portions (1a, 1b) are interconnected by a bridge of material (1c) presenting a flat (12) on the outside, and in that the bottom (13) of the slot extends parallel to the plane of the flat.

5. An assembly piece according to any one of claims 1 to 4, characterized in that the screw (3) includes a screw head (14) for coming into axial abutment against a shoulder formed in one of the portions (1a), and in that the screw includes a thread (4) for engaging in tapping (7) formed in the other portion (1b),

6. An assembly piece according to claim 5, characterized in that the screw extends along an axis (V) that is perpendicular to the slot (2).

## Patentansprüche

1. Verbindungsteil für das Zusammenfügen von zwei Stangen (T₁,T₂) einer ostheosynthetischen Vorrichtung, insbesondere bei der Ostheosynthese des Rückrats,
**dadurch gekennzeichnet, daß**
es folgendes aufweist:
- einen Körper (1) aus einem biologisch verträglichen Material mit einer allgemein abgerundeten Form, der einen Schlitz (2) aufweist, welcher zwei Abschnitte (1a, 1b) des Körpers unterteilt, sowie zwei Gewindebohrungen (5, 6) mit transversalen Achsen (A, B), welche den Schlitz in Querrichtung schneiden, und daß diese Gewindebohrungen (5, 6) jeweils eine der zu verbindenden Stangen aufnehmen können,
- sowie eine Stellschraube (3), welche die Abschnitte so verbindet, daß durch Festziehen der Schraube die beiden Stangen zwischen den Abschnitten (1a, 1b) eingeklemmt werden.

2. Verbindungsteil nach Anspruch 1,
**dadurch gekennzeichnet, daß**
der Körper (1) die allgemeine Form einer Kugel hat.

3. Verbindungsteil nach einem der Ansprüche 1 oder 2,
**dadurch gekennzeichnet, daß**
die Achsen (A, B) orthogonal verlaufen.

4. Verbindungsteil nach einem der Ansprüche 1 bis 3,
**dadurch gekennzeichnet, daß**
die Abschnitte (1a, 1b) untereinander mit Hilfe einer Materialbrücke (1c) verbunden sind, welche an ihrer Außenseite eine Abflachung (12) aufweist und dadurch, daß der Boden (13) des Schlitzes parallel zur Ebene dieser Abflachung verläuft.

5. Verbindungsteil nach einem der Ansprüche 1 bis 4,
**dadurch gekennzeichnet, daß**
die Schraube (3) einen Schraubenkopf (14) aufweist, welcher axial an einer Schulter anliegt, die in einen der Abschnitte (1a) eingebracht ist, und dadurch, daß die Schraube ein Außengewinde (4) aufweist, welches in ein Innengewinde (7) eingreifen kann, das in den anderen Abschnitt (1b) eingebracht ist.

6. Verbindungsteil nach Anspruch 5,
**dadurch gekennzeichnet, daß**
sich die Schraube entlang einer Achse (V) erstreckt, welche senkrecht zu dem Schlitz (2) ausgerichtet ist.
